# EUROPÄISCHE PATENTSCHRIFT

(11) **EP 2 197 870 B1**
(45) Veröffentlichungstag und Bekanntmachung des Hinweises auf die Patenterteilung: **13.07.2011**
(21) Anmeldenummer: 08804546.3
(22) Anmeldetag: 22.09.2008
(51) Int. Cl.: C07D 323/06, B01D 3/14

(54) **VERFAHREN ZUR ABTRENNUNG VON TRIOXAN AUS EINEM TRIOXAN/FORMALDEHYD/WASSER-GEMISCH MITTELS DRUCKWECHSEL-REKTIFIKATION**
METHOD FOR SEPARATING TRIOXANE FROM A TRIOXANE/FORMALDEHYDE/WATER MIXTURE BY MEANS OF PRESSURE CHANGE RECTIFICATION
PROCÉDÉ DE SÉPARATION DE TRIOXANE D'UN MÉLANGE DE TRIOXANE, DE FORMALDÉHYDE ET D'EAU, PAR RECTIFICATION SOUS PRESSION ALTERNÉE

(30) Priorität: 09.10.2007 EP 07118103
(43) Veröffentlichungstag der Anmeldung: 23.06.2010
(73) Patentinhaber: BASF SE, 67056 Ludwigshafen (DE)
(72) Erfinder: SIEGERT, Markus, 69126 Heidelberg (DE); LANG, Neven, 68163 Mannheim (DE); SZARVAS, Laszlo, 67071 Ludwigshafen (DE); SIGWART, Christoph, 69469 Weinheim (DE); WINDLIN, Franz Niklaus, 69120 Heidelberg (DE); STRÖFER, Eckhard, 68163 Mannheim (DE)
(86) Internationale Anmeldenummer: PCT/EP2008/062620
(87) Internationale Veröffentlichungsnummer: WO 2009/047109

(56) Entgegenhaltungen:
- WO-A-2005/063733
- WO-A-2008/090169
- WO-A2-2009/027434

## Beschreibung

Die Erfindung betrifft ein Verfahren zur Abtrennung von Trioxan aus einem Trioxan/Formaldehyd/Wasser-Gemisch sowie ein Verfahren zur Herstellung von Trioxan mit verbesserter Verfahrensausbeute bzw. verbesserter Produktreinheit.

Trioxan wird in der Regel durch Destillation von wässriger Formaldehydlösung in Gegenwart saurer Katalysatoren hergestellt. Dem Formaldehyd und Wasser enthaltenden Destillat wird anschließend das Trioxan durch Extraktion mit halogenierten Kohlenwasserstoffen, wie Methylenchlorid oder 1,2-Dichlorethan, oder anderen, mit Wasser nicht mischbaren Lösungsmitteln entzogen.

DE-A 1 668 867 beschreibt ein Verfahren zur Abtrennung von Trioxan aus Wasser, Formaldehyd und Trioxan enthaltenden Gemischen durch Extraktion mit einem organischen Lösungsmittel. Dabei wird eine aus zwei Teilstrecken bestehende Extraktionsstrecke an einem Ende mit einem üblichen organischen, mit Wasser praktisch nicht mischbaren Extraktionsmittel für Trioxan beschickt, am anderen Ende mit Wasser. Zwischen den beiden Teilstrecken wird das zu trennende Destillat der Trioxan-Synthese zugeführt. Auf der Seite der Lösungsmittelzuführung wird dann eine wässrige Formaldehydlösung und auf der Seite der Wasserzuführung eine praktisch formaldehydfreie Lösung von Trioxan in dem Lösungsmittel erhalten. In einem Beispiel wird das bei der Trioxan-Synthese entstandene Destillat aus 40 Gew.-% Wasser, 35 Gew.-% Trioxan und 25 Gew.-% Formaldehyd in den Mittelteil einer Pulsationskolonne eindosiert, am oberen Kolonnenende Methylenchlorid und am unteren Kolonnenende Wasser zugeführt. Dabei wird am unteren Kolonnenende eine etwa 25 gew.-%ige Lösung von Trioxan in Methylenchlorid und am oberen Kolonnenende eine etwa 30 gew.-%ige wässrige Formaldehydlösung erhalten.

Nachteil dieser Verfahrensweise ist der Anfall an Extraktionsmittel, welches aufgereinigt werden muss. Bei den verwendeten Extraktionsmitteln handelt es sich zum Teil um Gefahrenstoffe (T oder T⁺-Stoffe im Sinne der deutschen Gefahrstoffverordnung), deren Handhabung besondere Vorsichtsmaßnahmen erfordert.

DE-A 197 32 291 beschreibt ein Verfahren zur Abtrennung von Trioxan aus einem wässrigen Gemisch, das im Wesentlichen aus Trioxan, Wasser und Formaldehyd besteht, bei dem man dem Gemisch Trioxan durch Pervaporation entzieht und das an Trioxan angereicherte Permeat durch Rektifikation in Trioxan und ein azeotropes Gemisch aus Trioxan, Wasser und Formaldehyd trennt. In dem Beispiel wird ein wässriges Gemisch bestehend aus 40 Gew.-% Trioxan, 40 Gew.-% Wasser und 20 Gew.-% Formaldehyd in einer ersten Destillationskolonne unter Normaldruck in ein Wasser/Formaldehyd-Gemisch und in ein azeotropes Trioxan/Wasser/Formaldehyd-Gemisch getrennt. Das azeotrope Gemisch wird in eine Pervaporationseinheit geleitet, welche eine Membran aus Polydimethylsiloxan mit einem hydrophoben Zeolithen enthält. Das mit Trioxan angereicherte Gemisch wird in einer zweiten Destillationskolonne unter Normaldruck in Trioxan und wiederum in ein azeotropes Gemisch aus Trioxan, Wasser und Formaldehyd aufgetrennt. Dieses azeotrope Gemisch wird vor die Pervaporationsstufe zurückgeführt.

Nachteilig an dieser Verfahrensweise sind die sehr hohen Investitionen für die Pervaporationseinheit.

Die prioritätsältere, nicht vorveröffentlichte DE-A-07 101 198 beschreibt ein Verfahren zur Abtrennung von Trioxan aus einem Einsatzstrom I aus Formaldehyd, Trioxan und Wasser, bei dem
a) ein Einsatzstrom I, der als Hauptkomponente Formaldehyd und als Nebenkomponenten Trioxan und Wasser enthält, bereitgestellt wird,
b) der Einsatzstrom I, ein Rückführstrom V und ein Rückführstrom VII, der als Hauptkomponente Formaldehyd und als Nebenkomponenten Wasser und Trioxan enthält, in eine erste Destillationsstufe eingespeist und bei einem Druck von 0,1 bis 2,5 bar destilliert werden, wobei ein Strom II, der als Hauptkomponente Formaldehyd und als Nebenkomponente Wasser enthält, und ein Strom III, der als Hauptkomponente Trioxan und als Nebenkomponenten Wasser und Formaldehyd enthält, und ein Strom X, der Wasser, Trioxan und Formaldehyd enthält, erhalten werden,
c) der Strom III, gegebenenfalls nach Abtrennung von Leichtsiedern aus dem Strom III in einer Leichtsieder-Abtrennstufe, in einer zweiten Destillationsstufe bei einem Druck von 0,2 bis 17,5 bar destilliert wird, wobei der Druck in der zweiten Destillationsstufe um 0,1 bis 15 bar höher als der Druck in der ersten Destillationsstufe ist, wobei ein Strom IV, der im Wesentlichen aus Trioxan besteht, und der Rückführstrom V, der als Hauptkomponente Trioxan und als Nebenkomponenten Wasser und Formaldehyd enthält, erhalten wird,
d) der Strom X und gegebenenfalls ein Strom IX, der als Hauptkomponente Wasser enthält, in eine dritte Destillationsstufe eingespeist und bei einem Druck von d) der Strom X und gegebenenfalls ein Strom IX, der als Hauptkomponente Wasser enthält, in eine dritte Destillationsstufe eingespeist und bei einem Druck von 1 bis 10 bar destilliert wird, wobei ein Strom VI, der im Wesentlichen aus Wasser besteht, und ein Rückführstrom VII, der Formaldehyd und Wasser und Trioxan enthält, erhalten werden.

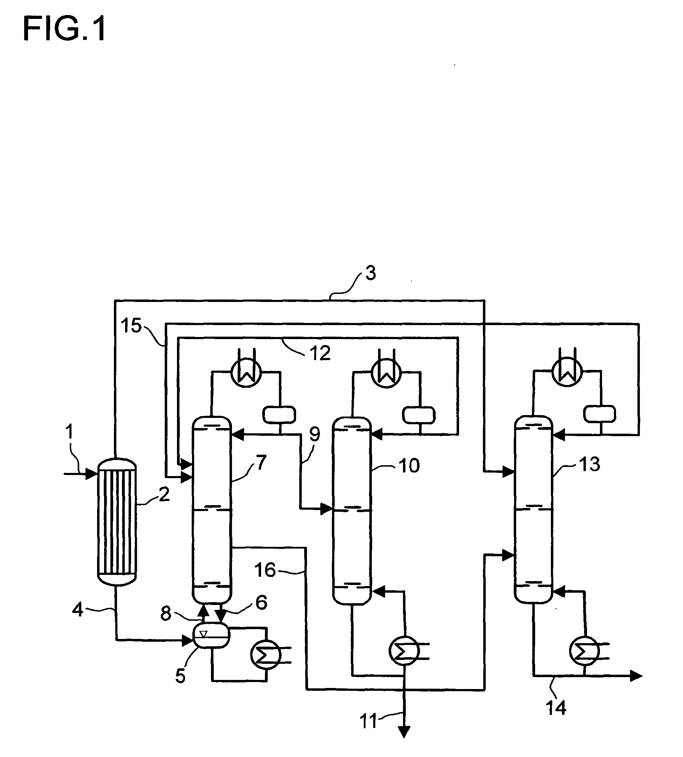

Die WO 2005/063733 betrifft ein Verfahren zur Abtrennung von Trioxan aus einem Trioxan/Formaldehyd/Wasser-Gemisch mittels Druckwechsel-Rektifikation. Wie in Figur 1 dargestellt, wird eine Rektifikation unter Einsatz dreier Kolonnen durchgeführt. Im dort beschriebenen Verfahren ist in der Kolonne 10 der Kopfstrom über die Leitung 12 mit der Mitte der dritten Kolonne 13 verbunden. Die Zuführungen 3 und 16 zur letzten Kolonne liegen auf gleicher Höhe.

Aufgabe der Erfindung ist es, ein Verfahren zur Abtrennung von Trioxan aus azeotropen Trioxan/Formaldehyd/Wasser-Gemischen bereitzustellen, welches ohne die Extraktionsschritte oder Pervaporationsschritte des Standes der Technik auskommt, Trioxan in höherer Reinheit bereitstellt und vorzugsweise eine verbesserte Verfahrensausbeute erlaubt.

Die Aufgabe wird erfindungsgemäß gelöst durch ein Verfahren zur Abtrennung von Trioxan aus einem Einsatzstrom I aus Formaldehyd, Trioxan und Wasser, bei dem
a) ein Einsatzstrom I, der als Hauptkomponente Formaldehyd und als Nebenkomponenten Trioxan und Wasser enthält, bereitgestellt wird,
b) der Einsatzstrom I, ein Rückführstrom V und ein Rückführstrom VII, der als Hauptkomponente Formaldehyd und als Nebenkomponenten Wasser und Trioxan enthält, in eine erste Destillationsstufe eingespeist und bei einem Druck von 0,1 bis 2,5 bar destilliert werden, wobei ein Strom II, der als Hauptkomponente Formaldehyd und als Nebenkomponente Wasser enthält, und ein Strom III, der als Hauptkomponente Trioxan und als Nebenkomponenten Wasser und Formaldehyd enthält, und ein Strom X, der Wasser, Trioxan und Formaldehyd enthält, erhalten werden,
c) der Strom III, gegebenenfalls nach Abtrennung von Leichtsiedern aus dem Strom III in einer Leichtsieder-Abtrennstufe, in einer zweiten Destillationsstufe bei einem Druck von 0,2 bis 17,5 bar destilliert wird, wobei der Druck in der zweiten Destillationsstufe um 0,1 bis 15 bar höher als der Druck in der ersten Destillationsstufe ist, wobei ein Strom IV, der im Wesentlichen aus Trioxan besteht, und der Rückführstrom V, der als Hauptkomponente Trioxan und als Nebenkomponenten Wasser und Formaldehyd enthält, erhalten wird,
c1) der Strom IV in mindestens einer weiteren Trioxan-Destillationsstufe bei einem Kopfdruck von 0,5 bis 2 bar aufgereinigt wird, wobei aufgereinigtes Trioxan als Seitenabzugsstrom XII im Verstärkungsteil der Kolonne gewonnen wird,
d) der Strom X und gegebenenfalls ein Strom IX, der als Hauptkomponente Wasser enthält und bei der Aufkonzentrierung von wässriger Formaldehydlösung erhalten wird, in eine dritte Destillationsstufe eingespeist und bei einem Druck von 1 bis 10 bar destilliert wird, wobei ein Strom VI, der im Wesentlichen aus Wasser besteht, und ein Rückführstrom VII, der als Hauptkomponente Formaldehyd und als Nebenkomponenten Wasser und Trioxan enthält, erhalten werden.

Es wurde erfindungsgemäß gefunden, dass es besonders vorteilhaft ist, den Strom IV nochmals in mindestens einer weiteren Trioxan-Destillationsstufe bei einem Kopfdruck von 0,5 bis 2 bar aufzureinigen. Dabei fällt aufgereinigtes Trioxan als Seitenabzugsstrom im Verstärkungsteil der Kolonne an. Besonders bevorzugt wird diese Destillation wiederum zweistufig durchgeführt, wobei der Seitenabzugsstrom der ersten Kolonne in den Sumpf der zweiten Kolonne geführt wird und hochreines Trioxan aus einem Seitenabzugsstrom der zweiten Kolonne entnommen wird.

Die Verfahrensausbeute kann zudem verbessert werden, indem man durch Zusatz von tertiären Aminen und/oder Iminen im Produktgemisch enthaltene Ameisensäure binden und das gebildete Ameisensäure-Amin-Salz in einer Flüssigphase (ionische Flüssigkeit) in einem Destillationssumpf ausschleusen kann. Diese Verfahrensweise wird weiter unten näher erläutert.

Die Hauptkomponente ist die Komponente mit dem größeren beziehungsweise größten Massenanteil an dem betreffenden Gemisch. Vorzugsweise beträgt der Massenanteil der Hauptkomponente an dem jeweiligen Gemisch mindestens 40 Gew.-%. Ein Strom "besteht im Wesentlichen aus" einer oder mehreren Komponenten, wenn er zu mindestens 90 Gew.-% aus dieser beziehungsweise diesen Komponenten besteht.

Es ist bekannt, dass Trioxan, Formaldehyd und Wasser ein ternäres Azeotrop bilden, welches bei einem Druck von 1 bar die Zusammensetzung 69,5 Gew.-% Trioxan, 5,4 Gew.-% Formaldehyd und 25,1 Gew.-% Wasser aufweist.

Erfindungsgemäß wird dieses Azeotrop durch Druckwechseldestillation umgangen, bei dem eine erste und eine zweite Destillation bei verschiedenen Drücken durchgeführt werden. In einer ersten Destillationskolonne, welche bei niedrigerem Druck betrieben wird, wird das Ausgangsgemisch in ein Trioxan/Wasser-Gemisch mit geringem Formaldehyd-Gehalt III und ein im Wesentlichen trioxanfreies Formaldehyd/WasserGemisch II aufgetrennt. Das Formaldehyd/Wasser-Gemisch II kann in die Trioxan-Synthese zurückgeführt werden. In einer zweiten, bei höherem Druck betriebenen Destillationskolonne wird das erhaltene Trioxan/Formaldehyd/Wasser-Gemisch III in reines Trioxan und ein Trioxan/Formaldehyd/Wasser-Gemisch V mit niedrigerem Trioxan-Gehalt aufgetrennt. Das Gemisch V wird in die erste Destillationskolonne zurückgeDestillationskolonne im Wesentlichen reines Wasser VI abgetrennt und ein wasserärmeres Trioxan/Formaldehyd/Wasser-Gemisch VII gewonnen wird. Dieses Gemisch VII wird in die erste Destillationskolonne zurückgeführt. Vorzugsweise wird der Destillationskolonne ein wasserhaltiger Strom IX, der bei der Aufkonzentrierung von wässriger Formaldehydlösung erhalten wird, ebenfalls in die dritte Destillationskolonne eingespeist.

Als Destillationskolonnen sind beliebige Destillationskolonnen wie Packungs- und Bodenkolonnen geeignet. Diese können beliebige Einbauten, Packungen oder Füllkörperschüttungen enthalten.

Der Druck in der zweiten Destillationsstufe ist um 0,1 bis 15 bar höher als der Druck in der ersten Destillationsstufe. Vorzugsweise beträgt diese Druckdifferenz 1,0 bis 10 bar, besonders bevorzugt 1,5 bis 5 bar.

Alle Druckangaben beziehen sich auf den Druck am Kopf der jeweiligen Kolonne.

Die erste Destillationsstufe wird bei einem Druck von 0,1 bis 2,5 bar, vorzugsweise 0,25 bis 1,5 bar durchgeführt. Die erste Destillationsstufe wird im Allgemeinen in einer Destillationskolonne mit mindestens 2, vorzugsweise 2 bis 50, besonders bevorzugt 4 bis 25 theoretischen Stufen durchgeführt. Im Allgemeinen umfasst der Abtriebsteil dieser Kolonne mindestens 25 %, vorzugsweise 50 bis 90 % der theoretischen Stufen dieser Kolonne.

Der Einspeisungsstrom I enthält im Allgemeinen 40 bis 80 Gew.-% Formaldehyd, 20 bis 59 Gew.-% Wasser und 1,0 bis 30 Gew.-% Trioxan. Der Einspeisungsstrom I wird vorzugsweise dampfförmig in den Sumpf der ersten Destillationskolonne eingespeist.

Der Strom II, der im Allgemeinen als Sumpfabzugsstrom der ersten Destillationskolonne erhalten wird, enthält im Allgemeinen weniger als 5 Gew.-%, bevorzugt weniger als 2 Gew.-% Trioxan, besonders bevorzugt weniger als 1 Gew.-% Trioxan. Beispielsweise setzt sich der Strom II wie folgt zusammen: 55 bis 85 Gew.-% Formaldehyd, 15 bis 45 Gew.-% Wasser und 0 bis 5 Gew.-% Trioxan. Der Strom III, der im Allgemeinen als Kopfabzugsstrom der ersten Destillationskolonne erhalten wird, enthält im Allgemeinen mehr als 60 Gew.-%, vorzugsweise mehr als 63 Gew.-%, besonders bevorzugt mehr als 65 Gew.-% Trioxan. Beispielsweise setzt sich der Strom III wie folgt zusammen: 3 bis 20 Gew.-% Formaldehyd, 10 bis 30 Gew.-% Wasser und 60 bis 75 Gew.-% Trioxan. Der Strom X, der als Seitenabzugsstrom der ersten Destillationskolonne erhalten wird, enthält Wasser, Formaldehyd und Trioxan, wobei im Allgemeinen Wasser oder Formaldehyd die Hauptkomponente ist. Beispielsweise setzt sich der Strom X wie folgt zusammen: 10 bis 50 Gew.-% Formaldehyd, 10 bis 50 Gew.-% Wasser und 3 bis 40 Gew.-% Trioxan. -

Der Strom II wird vorzugsweise in die Trioxan-Synthese zurückgeführt.

Die Ströme I, III, V und VII können noch bis zu 15 Gew.-% Leichtsieder enthalten. Übliche Leichtsieder, die bei der Trioxan-Synthese und der nachfolgenden destillativen Trennung gebildet werden können, sind Methylformiat, Methylal, Dimethoxydimethylether, Methanol, Ameisensäure sowie weitere Halb- und Vollacetale. Zur Abtrennung dieser Leichtsieder kann optional zwischen der ersten und der zweiten Destillationsstufe eine Leichtsieder-Abtrennstufe durchgeführt werden. Dabei werden die Leichtsieder vorzugsweise über den Kopf einer Leichtsieder-Abtrennkolonne, welche im Allgemeinen bei einem Druck von 0,1 bis 5 bar, vorzugsweise bei einem Druck von 1,0 bis 2,5 bar betrieben wird, abgetrennt. Im Allgemeinen weist die Leichtsieder-Abtrennkolonne mindestens 2 theoretische Stufen, vorzugsweise 15 bis 50 theoretische Stufen auf. Im Allgemeinen umfasst der Abtriebsteil dieser Kolonne 25 bis 90 %, vorzugsweise 50 bis 75 % der theoretischen Stufen dieser Kolonne. Der Gehalt der gegenüber Trioxan leichter siedenden Komponenten im Sumpfaustrag der Leichtsieder-Abtrennkolonne beträgt im Allgemeinen weniger als 5 Gew.-%, bevorzugt weniger als 2,5 Gew.-%, besonders bevorzugt weniger als 1,5 Gew.-%.

Im Allgemeinen wird eine Leichtsieder-Abtrennung durchgeführt.

Der Strom III wird in einer zweiten Destillationsstufe bei einem Druck von 0,2 bis 17,5 bar in einen Strom IV aus im Wesentlichen reinem Trioxan und einen Strom V, der als Hauptkomponente Trioxan und daneben Wasser und Formaldehyd enthält, aufgetrennt. Diese zweite Destillationsstufe wird vorzugsweise bei 2,5 bis 10 bar durchgeführt. Im Allgemeinen wird diese zweite Destillationsstufe in einer Destillationskolonne mit mindestens 2 theoretischen Böden, vorzugsweise 10 bis 50 theoretischen Böden, durchgeführt, wobei der Strom IV als Sumpfabzugsstrom oder als Seitenabzugsstrom im Abtriebsteil der Kolonne anfällt und der Strom V als Kopfabzugsstrom anfällt. Im Allgemeinen umfasst der Abtriebsteil dieser Destillationskolonne 25 bis 90 %, vorzugsweise 50 bis 75 % der theoretischen Stufen dieser Kolonne.

Im Allgemeinen enthält der Strom IV 95 bis 100 Gew.-%, vorzugsweise 99 bis 100 Gew.-% Trioxan und 0 bis 5 Gew.-%, vorzugsweise 0 bis 1 Gew.-% Wasser und Nebenkomponenten. Nebenkomponenten sind insbesondere die oben genannten Leichtsieder, aber auch höher als Trioxan siedende Komponenten. Besonders bevorzugt ist der Gehalt an Wasser und Nebenkomponenten im Trioxan-Strom IV < 0,1 %. Er kann sogar < 0,01 % sein. Der Strom V enthält beispielsweise 5 bis 20 Gew.-% Formaldehyd, 15 bis 35 Gew.-% Wasser und 50 bis 75 Gew.-% Trioxan.

Erfindungsgemäß wird der Strom IV in mindestens einer weiteren Trioxan-Destillationsstufe bei einem Kopfdruck von 0,5 bis 2 bar aufgereinigt, wobei aufgereinigtes Trioxan als Seitenabzugsstrom XII im Verstärkungsteil der Kolonne gewonnen wird. Diese Destillationskolonne weist bevorzugt 5 bis 50, besonders bevorzugt 10 bis 20 theoretische Trennstufen auf. Der Kopfdruck der Kolonne beträgt vorzugsweise 1,0 bis 1,5 bar. Im Seitenabzugsstrom wird vorzugsweise Rein-Trioxan mit einer Reinheit > 99,9 Gew.-%, besonders bevorzugt > 99,99 Gew.-% gewonnen.

Auf diese Stufe c1) kann eine zweite Trioxan-Destillation des Stroms XII als Stufe c2) folgen. Diese Stufe wird ebenfalls bei einem Kopfdruck im Bereich von 0,5 bis 2,0 bar, besonders bevorzugt 1,0 bis 1,5 bar durchgeführt, wobei nochmals aufgereinigtes Trioxan als Seitenabzugsstrom im Verstärkungsteil der Kolonne gewonnen wird. Diese weitere Destillationskolonne weist ebenfalls bevorzugt 5 bis 50, besonders bevorzugt 10 bis 20 theoretischen Trennstufen auf. Als Seitenabzugsstrom wird polymerisationsfähiges Reinst-Trioxan gewonnen.

Der Strom X und gegebenenfalls ein wasserhaltiger Strom IX werden in einer dritten Destillationsstufe bei einem Druck von 1 bis 10 bar in einen Strom VI, der im Wesentlichen aus Wasser besteht, und einen Rückführstrom VII, der als Hauptkomponente Trioxan und daneben Wasser und Formaldehyd enthält, aufgetrennt. Der wasserhaltige Strom IX wird gegebenenfalls als Brüdenabzugsstrom einer Formaldehyd-Aufkonzentrierungseinheit, die als Verdampfer ausgestaltet ist, gewonnen und enthält beispielsweise 70 bis 97 Gew.-% Wasser und 3 bis 30 Gew.-% Formaldehyd. Vorzugsweise wird die dritte Destillationsstufe bei einem Druck von 2,5 bis 6,5 bar durchgeführt. Im Allgemeinen wird die dritte Destillationsstufe in einer Destillationskolonne mit mindestens 2 theoretischen Böden, vorzugsweise 10 bis 50 theoretischen Böden, durchgeführt, wobei der Wasserstrom VI als Sumpfabzugsstrom oder als Seitenabzugsstrom der Kolonne und der Rückführstrom VII als Kopfabzugsstrom erhalten werden. Der Strom X wird vorzugsweise im oberen Bereich der Kolonne, beispielsweise im Bereich des obersten Drittels der theoretischen Böden der Kolonne, und der Strom IX im mittleren Bereich der Kolonne, beispielsweise im Bereich des mittleren Drittels der theoretischen Böden der Kolonne, zugegeben.

Der Wasserstrom VI besteht vorzugsweise zu mehr als 95 Gew.-%, besonders bevorzugt zu mehr als 97 Gew.-% aus Wasser. Beispielsweise enthält der Strom VI 98 bis 100 Gew.-% Wasser, 0 bis 1 Gew.-% Formaldehyd und 0 bis 1 Gew.-% Nebenkomponenten.

Der Strom VII enthält beispielsweise 10 bis 55 Gew.-% Formaldehyd, 5 bis 50 Gew.-% Wasser und 5 bis 55 Gew.-% Trioxan.

Der Strom VII kann teilweise oder vollständig vor die erste Destillationsstufe zurückgeführt werden, vorzugsweise wird er im Wesentlichen vollständig in die erste Destillationsstufe zurückgeführt. Dabei kann er mit dem Rückführstrom V gemischt oder getrennt von diesem der ersten Destillationskolonne zugeführt werden.

Gegenstand der vorliegenden Erfindung ist auch ein Verfahren zur Herstellung von Trioxan aus einer wässrigen Formaldehydlösung, bei dem der Formaldehyd, Trioxan und Wasser enthaltende Einsatzstrom I in einer vorgelagerten Trioxan-Synthesestufe aus einer wässrigen Formaldehydlösung hergestellt wird und anschließend aus dem Strom I wie vorstehend beschrieben Trioxan abgetrennt wird.

Gegenstand der vorliegenden Erfindung ist auch ein Verfahren zur Herstellung von Trioxan aus einer wässrigen Formaldehydlösung, bei dem der Formaldehyd, Trioxan und Wasser enthaltende Einsatzstrom I in einer vorgelagerten Trioxan-Synthesestufe aus einer wässrigen Formaldehydlösung hergestellt wird und anschließend aus dem Strom I wie vorstehend beschrieben Trioxan abgetrennt wird. Alternativ dazu können die Trioxan-Synthese und die erste Destillationsstufe in einer Reaktivdestillation vereinigt werden.

In einer Ausführungsform des erfindungsgemäßen Verfahrens wird ein Strom XI aus einer wässrigen Formaldehydlösung einer vorgelagerten Trioxan-Synthesestufe zugeführt und in Gegenwart saurer homogen oder heterogen vorliegender Katalysatoren wie Ionenaustauscherharze, Zeolithe, Schwefelsäure und p-Toluolsulfonsäure bei einer Temperatur von im Allgemeinen 70 bis 130 °C umgesetzt. Dabei kann in einer Destillationskolonne oder einem Verdampfer (Reaktivverdampfer) gearbeitet werden. Das Produktgemisch aus Trioxan/Formaldehyd und Wasser fällt dann als dampfförmiger Brüdenabzugsstrom des Verdampfers beziehungsweise als Kopfabzugsstrom am Kopf der Kolonne an. Die Trioxan-Synthesestufe kann auch in einem Festbett- oder Fließbettreaktor an einem heterogenen Katalysator, z. B. einem Ionenaustauscherharz oder Zeolith, durchgeführt werden.

In einer weiteren Ausführungsform des erfindungsgemäßen Verfahrens werden die Trioxan-Synthesestufe und die erste Destillationsstufe als Reaktivdestillation in einer Reaktionskolonne durchgeführt. Diese kann im Abtriebsteil ein Katalysator-Festbett aus einem heterogenen sauren Katalysator enthalten. Alternativ kann die Reaktivdestillation auch in Gegenwart eines homogenen Katalysators durchgeführt werden, wobei der saure Katalysator zusammen mit der wässrigen Formaldehyd-Lösung im Kolonnensumpf vorliegt.

Im Allgemeinen enthält die wässrige Formaldehydlösung, die der Trioxan-Synthesestufe zugeführt wird, 30 bis 85 Gew.-% Formaldehyd und 15 bis 70 Gew.-% Wasser. Diese Lösung kann in einem vorgelagerten Aufkonzentrierungsschritt aus einer wässrigen Formaldehydlösung mit niedrigerer Formaldehyd-Konzentration erhalten werden. Der Aufkonzentrierungsschritt kann beispielsweise in einem Verdampfer, vorzugsweise einem Fallfilmverdampfer, durchgeführt werden.

Der vorgelagerte Aufkonzentrierungsschritt kann beispielsweise wie in DE-A 199 25 870 beschrieben durchgeführt werden.

In einer Ausführungsform des erfindungsgemäßen Verfahrens wird ein Strom VIII einer wässrigen Formaldehydlösung in einem Verdampfer, vorzugsweise einem Fallfilmverdampfer, aufkonzentriert, wobei der Strom XI aus wässriger Formaldehydlösung mit höherer Formaldehyd-Konzentration erhalten wird. Der Brüdenabzugsstrom des Verdampfers, welcher stark an Formaldehyd abgereichert ist, wird als wasserhaltiger Strom IX in die dritte Destillationsstufe eingespeist. Strom VIII enthält beispielsweise 40 bis 60 Gew.-% Formaldehyd und 40 bis 60 Gew.-% Wasser. Der aufkonzentrierte Strom XI enthält beispielsweise 55 bis 80 Gew.-% Formaldehyd und 20 bis 45 Gew.-% Wasser. Der formaldehydarme Brüdenabzugsstrom IX enthält beispielsweise 10 bis 25 Gew.-% Formaldehyd und 75 bis 90 Gew.-% Wasser.

Das erhaltene Rein-Trioxan, dessen Reinheit > 99 Gew.-%, > 99,9 Gew.-% oder sogar > 99,99 Gew.-% betragen kann, oder das polymerisationsfähige Reinst-Trioxan wird vorzugsweise zur Herstellung von Polyoxymethylen (POM), Polyoxymethylenderivaten wie Polyoxymethylendimethylether (POMDME) und Diaminodiphenylmethan (MDA) verwendet.

Die Erfindung betrifft zudem eine Verfahrensweise zur Abtrennung von Ameisensäure aus dem Formaldehyd, Trioxan, Wasser und Ameisensäure enthaltenden Gemisch durch Destillation. Hierdurch ist eine Verbesserung der Rohausbeute des Trioxanverfahrens möglich, und das Trioxan kann zusätzlich stabilisiert werden.

Schwierig ist in der Regel die Abtrennung von Ameisensäure, die beim Destillieren zusammen mit Trioxan übergeht. Die Ameisensäure entsteht beispielsweise durch Cannizzaro-Reaktion aus Formaldehyd, wobei jedenfalls ein Äquivalent Methanol entsteht. Da Ameisensäure die Zersetzung des Trioxans katalysieren kann, sind die effektive Abtrennung der Ameisensäure und dadurch die Unterdrückung der Trioxan-Zersetzung sehr wichtig. Wie beschrieben wird in einer unter Druck betriebenen Destillationskolonne ein Stoffgemisch mit annähernd azeotroper Zusammensetzung der Hauptkomponenten Trioxan, Formaldehyd und Wasser bei Temperaturen von bis zu oberhalb von 180°C in reines Trioxan im Sumpfaustrag und ein Azeotrop im Kopfaustrag getrennt. Aufgrund der gewählten Destillationsbedingungen enthält der Sumpfaustrag häufig größere Mengen an Ameisensäure, beispielsweise 5000 ppm Ameisensäure. Die Ameisensäure stört die Polymerisation von Trioxan zu Polyoximethylen (POM) jedoch stark und führt zudem zu einer Verschlechterung der POM-Qualität, so dass der maximale Ameisensäure-Gehalt stark vermindert werden sollte.

Dies wird erfindungsgemäß vorzugsweise dadurch erreicht, dass man dem Gemisch vor oder während der Destillation mindestens ein tertiäres Amin und/oder ein Imin oder ein Gemisch davon, das die Ameisensäure deprotonieren und in ein Salz überführen kann, in einer katalytischem Menge oder in einer Menge zusetzt, die zur Salzbildung mit der gesamten Ameisensäurenmenge ausreicht, und das gebildete Ameisensäure-Amin-Salz in einer Flüssigphase im Destillationssumpf ausschleust.

Es können somit einzelne Amine oder Imine, Gemische von Aminen oder Iminen oder auch Gemische von Aminen und Iminen eingesetzt werden.

Erfindungsgemäß wird bevorzugt ein tertiäres Amin oder ein Gemisch tertiärer Amine eingesetzt, das die Ameisensäure deprotonieren und in ein Salz überführen kann.

Der Ausdruck "tertiäres Amin" beschreibt eine stickstoffhaltige Verbindung, in der im Unterschied zu Ammoniak alle drei Wasserstoffatome durch organische Reste ersetzt sind. Dabei kann es sich um azyklische oder zyklische Strukturen handeln, wobei zyklische Strukturen aliphatisch oder aromatisch sein können, wie etwa im Falle von Pyridin. Bevorzugt ist das tertiäre Amin ausgewählt aus Tri-C₁₋₃-Alkylamin, cyclischen oder bicyclischen aliphatischen tertiären Aminen, Imidazol oder Pyridin. Dabei kann es sich um ein Trialkylamin handeln, sofern das tertiäre Amin nur ein Stickstoffatom aufweist. Es ist erfindungsgemäß auch möglich, dass das tertiäre Amin mehrere Stickstoffatome aufweist, in denen jeweils die Wasserstoffatome durch organische Reste ersetzt sind. Es ist zudem möglich, dass die organischen Reste, beispielsweise zusammen mit weiteren Heteroatomen wie Stickstoffatomen, bizyklische Strukturen bilden. Vorzugsweise weisen die erfindungsgemäß einsetzten tertiären Amine zwei oder drei, insbesondere zwei tertiäre Stickstoffatome auf. Besonders bevorzugt handelt es sich um eine Diazabicycloalkan-Verbindung oder Diazabicycloalken-Verbindung. Besonders bevorzugte Beispiele sind Diazabicycloundecen (DBU) und Triethylendiamin (TEDA, DABCO®).

Das tertiäre Amin hat eine so große Basizität, dass es die Ameisensäure deprotonieren und in ein Salz überführen kann. Das tertiäre Amin wird zudem so gewählt, dass das gebildete Ameisensäure-Amin-Salz in einer Flüssigphase (ionische Flüssigkeit) vorliegt. Zur entsprechenden Salzbildung ist das Molverhältnis von tertiären Stickstoffatomen zu Ameisensäure vorzugsweise im Bereich von 1 : 1 bis 3 : 1, besonders bevorzugt im Bereich von 1 : 1 bis 2 : 1, insbesondere etwa 1 : 1.

Die Basizität des Amins ist wichtig: Eine starke Base ist notwendig, um die Ameisensäure vollständig zu deprotonieren, womit in der Flüssigphase ein niedrig schmelzendes, stabiles Salz gebildet wird. Durch die Entfernung der Ameisensäure aus dem Produktgemisch wird die autokatalytische Zersetzung des Trioxans stark verringert. Durch das Vorliegen eines niedrig schmelzenden Salzes (ionische Flüssigkeit) kann ein Feststoff-Handling vermieden werden. Besonders vorteilhaft wird Triethylendiamin als Base einsetzt. Das Ammoniumsalz TEDA * HCOO kann als Schwersieder abgetrennt werden und lässt sich bei erhöhten Temperaturen in der Gasphase wieder zu TEDA und HCOOH bzw. CO und H₂O oder CO₂ und H₂ spalten. Dies ermöglicht einen katalytischen Einsatz des Amins durch Rückführung im Verfahren, d. h. das Amin (oder Imin) wird nicht verbraucht.

Da das tertiäre Amin eine stabilisierende Wirkung auf Trioxan ausübt, kann man sich zusätzlich diese Stabilisierungswirkung zunutze machen. In diesem Fall wird das Amin in einem molaren Überschuss gegenüber der Ameisensäure zugesetzt, so dass das Trioxan basisch stabilisiert wird. Der molare Überschuss beträgt vorzugsweise das 2- bis 5-fache, d. h. 2 : 1 bis 5 : 1.

Das Ameisensäure-Amin-Salz liegt vorzugsweise bei Umgebungstemperatur (25°C) als ionische Flüssigkeit vor.

Bei der Destillation wird das tertiäre Amin vorzugsweise in den Feed, Abtriebsteil, Verstärkerteil und/oder Sumpf mindestens einer Destillationskolonne zugegeben. Bevorzugt ist der Zusatz im Feed mindestens einer der Kolonnen in der zweiten und dritten Destillationsstufe. Bevorzugt ist der Zusatz in der zweiten Destillationsstufe.

Das Ameisensäure-Amin-Salz wird dabei mit dem Destillationssumpf ausgeschleust. Bevorzugt wird es sodann einer weiteren Destillation unterworfen, in der es aus dem Destillationssumpf ausgeschleust wird.

Das ausgeschleuste Ameisensäure-Amin-Salz kann unter Erwärmung zersetzt werden, wobei das tertiäre Amin zurückgewonnen wird, das sodann in das Verfahren zurückgeführt werden kann. Damit ist es möglich, das tertiäre Amin im Kreis zu fahren, ohne dass ein nennenswerter Verbrauch auftritt. Hierdurch wird das Verfahren besonders ökonomisch. Die Zersetzung des Ameisensäure-Amin-Salzes unter Erwärmung ist beispielsweise in Angew. Chem. 82, 1970, Nr. 2, Seiten 73 bis 77, beschrieben.

Die Erfindung wird nachfolgend mit Bezugnahme auf die Zeichnung näher erläutert.

Es zeigt:
- Figur 1: beispielhaft eine Ausführungsform des erfindungsgemäßen Verfahrens.

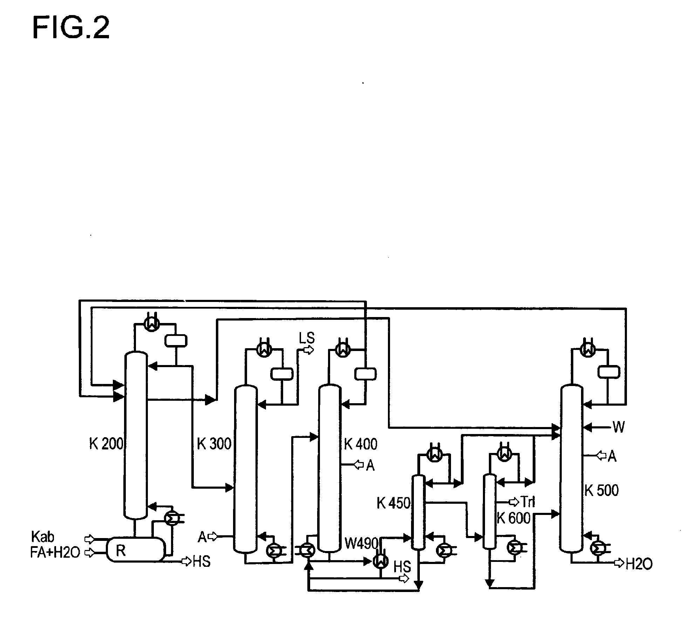

Eine wässrige Formaldehydlösung **1** (Strom VIII) wird dem Verdampfer **2,** beispielsweise einem Dünnschichtverdampfer, Fallfilmverdampfer oder Wendelrohrverdampfer, zugeführt. Als Brüdenabzugsstrom **3** (Strom IX) des Verdampfers wird eine an Formaldehyd abgereicherte wässrige Lösung, als Sumpfabzugsstrom **4** (Strom XI) des Verdampfers eine formaldehydreiche wässrige Lösung erhalten. Diese wird mit dem formaldehydreichen Sumpfabzugsstrom **6** (Strom II) der ersten Destillationskolonne **7** dem Trioxan-Synthesereaktor **5,** der als Verdampfer ausgebildet ist, zugeführt. Das den Trioxan-Synthesereaktor verlassende dampfförmige Trioxan/Formaldehyd/Wasser-Gemisch **8** (Strom I) wird dem Sumpf der ersten Destillationskolonne **7** zugeführt. Der trioxanreiche Kopfabzugsstrom **15** (Strom VII) der dritten Destillationskolonne **13** wird der Destillationskolonne **7** in der Nähe des Kolonnenkopfes zugeführt. Der Destillationskolonne **7** wird ein Formaldehyd/Wasser-Strom **6** (Strom II) als Sumpfabzugsstrom, ein wasserarmer Formaldehyd/Wasser/Trioxan-Strom **9** (Strom III) als Kopfabzugsstrom und ein wasserreicher Formaldehyd/Wasser/Trioxan-Strom **16** als Seitenabzugsstrom entnommen. Strom **6** wird zusammen mit dem Strom **4** in den Reaktor **5** zurückgeführt. Der wasserarme Formaldehyd/Wasser/Trioxan-Strom **9** wird der Destillationskolonne **10** zugeführt und dort in einen Sumpfabzugsstrom **11** (Strom IV) aus im Wesentlichen reinem Trioxan und einen Kopfabzugsstrom **12** (Strom V), der überwiegend Trioxan und daneben Wasser und Formaldehyd enthält, aufgetrennt. Der Strom **12** wird in die erste Destillationskolonne zurückgeführt. Der wasserreiche Formaldehyd/Wasser/Trioxan-Strom **16** und der formaldehydarme wässrige Brüdenabzugsstrom **3** (Strom IX) des Verdampfers **2** werden der dritten Destillationskolonne zugeführt und dort in einen Strom **14** (Strom VI), der im Wesentlichen aus Wasser besteht und ausgeschleust wird, und den Rückführstrom **15** (Strom VII), der überwiegend Formaldehyd und daneben Wasser und Trioxan enthält, aufgetrennt. Der Strom IV wird einer oder zwei weiteren Destillationsstufen zugeführt, wie in Figur 2 dargestellt.

### Beispiel

Bei der rechnerischen Simulation des in der Figur dargestellten Verfahrens wurden Stoffströme 4, 9, 11, 12, 3, 14, 15 und 16 der in den Tabellen angegebenen Zusammensetzungen erhalten. Dabei wurden folgende Parameter gewählt: Die erste Destillationsstufe wird bei einem Druck von 0,7 bar in einer Kolonne 7 mit 10 theoretischen Böden durchgeführt. Das Rücklaufverhältnis beträgt 0,8, die Kopftemperatur 80 °C und die Sumpftemperatur 94°C. Die zweite Destillationsstufe wird bei einem Druck von 4,0 bar in einer Kolonne **10** mit 40 theoretischen Böden durchgeführt. Das Rücklaufverhältnis beträgt 0,5, die Kopftemperatur 146 °C und die Sumpftemperatur 181 °C. Der Zulauf **9** befindet sich auf Höhe des 35. theoretischen Bodens. Die dritte Destillationsstufe wird bei einem Druck von 6,0 bar in einer Kolonne **13** mit 10 theoretischen Böden durchgeführt. Das Rücklaufverhältnis beträgt 1,5, die Kopftemperatur 146 °C und die Sumpftemperatur 160 °C. Der Zulauf **3** befindet sich auf Höhe des 8. theoretischen Bodens.

| Strom | 4 (XI) | 9 (III) | 11 (IV) | 12 (V) | 3 (IX) | 14 (VI) | 15 (VII) | 16 (X) |
|---|---|---|---|---|---|---|---|---|
| Mengenstrom [kg/h] | 4,1 | 11,9 | 3 | 9,0 | 2,0 | 3,1 | 8,3 | 9,5 |
| Formaldehyd [Gew.-%] | 65,0 | 8,5 | < 1 | 11,3 | 15,3 | < 1 | 52,2 | 42,7 |
| Wasser [Gew.-] | 35,0 | 21,5 | < 1 | 28,7 | 84,7 | > 99 | 22,6 | 35,2 |
| Trioxan [Gew.-%] | 0 | 70,0 | > 99 | 60,0 | 0 | 0 | 25,2 | 22,1 |

Figur 2 zeigt beispielhaft eine Ausführungsform des erfindungsgemäßen Verfahrens, wobei auch die weiteren Destillationsstufen K450 und K600 gezeigt sind. In der Figur bedeuten:
- Kat: Katalysator
- FA: Formaldehyd
- R: Reaktor
- HS: Hochsieder
- A: Amin-Einspeisung
- Tri: Trioxan
- LS: Leichtsieder
- W: wasserhaltiger Strom

Im Bereich von Reaktor R und Kolonne K200 werden die Aufkonzentrierung und Reaktion durchgeführt. Die Leichtsieder-Abtrennung erfolgt in der Kolonne K300. Es schließt sich die Trioxan-Abtrennung in den Kolonnen K400, K450 und K600 an. Die Wasserabtrennung erfolgt in der Kolonne K500.

In dieser Ausführungsform sind die Reaktion und Aufkonzentrierung apparativ getrennt. Alternativ können auch Reaktion und Aufkonzentrierung in der Kolonne K200 apparativ vereint werden, so dass Katalysator, Formaldehyd und Wasser am Boden der Reaktiv-Destillationskolonne zugesetzt werden, während Hochsieder aus dem Kolonnensumpf ausgeschleust werden.

Die Destillationskolonnen können so verschaltet werden, dass der bei der Trioxan-synthese anfallende Reaktoraustrag der ersten Destillationskolonne K200 zugeführt wird, deren Sumpfabzug, falls Trioxan-Synthese und Trioxan-Aufkonzentrierung apparativ getrennt sind, siehe Figur 2, in die Trioxan-Synthesestufe eingespeist wird. Ansonsten, falls die Trioxan-Synthese im Sumpfbereich der Kolonne stattfindet, wird der Sumpfabzug üblicherweise dem Sumpfverdampfer der Kolonne zugeführt. Das trioxanreiche Destillat der ersten Kolonne wird in eine zweite Destillationskolonne K300 eingespeist, in welcher sämtliche in den folgenden Aufarbeitungsschritten störenden und gegenüber Trioxan leichter siedenden Komponenten über Kopf abgetrennt werden, und der Sumpfaustrag der zweiten Kolonne wird in eine dritte Destillationskolonne K400 eingespeist, in welcher über einen Seitenabzugsstrom im Abtriebsteil oder direkt am Sumpfabzug Roh-Trioxan gewonnen wird. Der Seitenabzugsstrom bzw. der Sumpfaustrag der K400 wird einer vierten Kolonne K450 in den Abtriebsteil, bevorzugt in den Sumpf zugeführt, und das am Seitenabzug im Verstärkungsteil der Kolonne K450 gewonnene Rein-Trioxan wird der fünften Kolonne K600 eingespeist, in welcher an einem Seitenabzug im Verstärkungsteil polymerisationsfähiges Reinst-Trioxan anfällt. Das Kopfprodukt der K400 wird bevorzugt der sechsten Kolonne K500 und besonders bevorzugt der ersten Kolonnen K200 sowie der Seitenabzug der K200, eventuell noch durch Zumischung eines weiteren wasserhaltigen Stoffstroms, welcher beispielsweise aus der Aufkonzentrierung eines formaldehydhaltigen Stoffstroms resultiert, anschließend der sechsten Kolonne K500 zur Gewinnung eines wasserhaltigen Stroms zugeführt, in welcher reines Wasser über einen Seitenabzugsstrom im Abtriebsteil oder direkt am Sumpfabzug der Kolonne gewonnen wird. Das Kopfprodukt wird zur ersten Kolonne K200 zurückgeführt.

Der Sumpfaustrag der vierten Kolonne K450 kann zur sechsten Kolonne K500, oder bevorzugt zur zweiten Kolonne K300, oder besonders bevorzugt zur dritten Kolonne K400 zurückgeführt werden.

Der Kopfaustrag der vierten Kolonne K450 kann zur dritten Kolonne K400, oder bevorzugt zur zweiten Kolonne K300, oder besonders bevorzugt zur sechsten Kolonne K500 zurückgeführt werden.

Der Sumpfaustrag der fünften Kolonne K600 kann zur dritten Kolonne K400, oder bevorzugt zur zweiten Kolonne K300, oder besonders bevorzugt zur sechsten Kolonne K500 zurückgeführt werden.

Der Kopfaustrag der vierten Kolonne K450 kann zur dritten Kolonne K400, oder bevorzugt zur zweiten Kolonne K300, oder besonders bevorzugt zur sechsten Kolonne K500 zurückgeführt werden.

Das über Sumpf oder über einen Seitenabzug im Abtriebsteil abgezogene Roh-Trioxan kann in der dritten Kolonne K400 ein- oder mehrstufig verdampft werden, siehe W490 in Figur 2, so dass die über die Zugabe des Amins im Rohtrioxan existierenden Hochsieder, wie z. B. Formiate, abgetrennt werden, bevor der an hochsiedenden Komponenten verarmte Strom, gasförmig oder kondensiert, in den Abtriebsteil, bevorzugt direkt in den Sumpfbereich, der vierten Kolonne K450 eingespeist wird.

Der mit hochsiedenden Komponenten angereicherte Austrag der einstufigen bzw. mehrstufigen Verdampfung kann bis auf einen geringen Ausschleusstrom, welcher der sechsten Kolonne K500 zugeführt oder verworfen wird, zur dritten Kolonne K400 zurückgeführt und in den Abtriebsteil, bevorzugt in den Sumpf der Kolonne eingespeist werden.

Der Verstärkungsteil der Destillationskolonne K200 kann zur Anreicherung des in der Synthese gebildeten Trioxans 10 bis 100%, bevorzugt 50 bis 100% der theoretischen Trennstufen der Kolonne aufweisen.

Der Verstärkungsteil der Destillationskolonne K300 kann zur Abtrennung der gegenüber dem Trioxan im Reaktoraustrag enthaltenden leichter siedenden Komponenten 25 bis 95%, bevorzugt 50 bis 75% der theoretischen Trennstufen der Kolonne aufweisen.

Der Abtriebsteil der Destillationskolonne K400 kann zur Gewinnung von Roh-Trioxan 25 bis 100%, bevorzugt 75 bis 100% und besonders bevorzugt 90 bis 100% der theoretischen Trennstufen der Kolonne aufweisen.

Der Verstärkungsteil der Destillationskolonne K450 kann zur Gewinnung von Rein-Trioxan 25 bis 100%, bevorzugt 75 bis 100% und besonders bevorzugt 90 bis 100% der theoretischen Trennstufen der Kolonne aufweisen.

Der Verstärkungsteil der Destillationskolonne K600 kann zur Gewinnung von polymerisationsfähigem Reinst-Trioxan 25 bis 100%, bevorzugt 75 bis 100% und besonders bevorzugt 90 bis 100% der theoretischen Trennstufen der Kolonne aufweisen.

Der Abtriebsteil der Destillationskolonne K500 kann zur Gewinnung eines wasserhaltigen Stroms 25 bis 100, bevorzugt 75 bis 100% und besonders bevorzugt 90 bis 100% der theoretischen Trennstufen der Kolonne aufweisen.

Die Destillationskolonnen können mit geordneten Packungen, Füllkörpern oder Böden bestückt und thermisch gekoppelt sein.

Die thermisch gekoppelten Destillationskolonnen können jeweils mit einem eigenen Verdampfer und Kondensator ausgestattet sein.

Die beiden thermisch gekoppelten Kolonnen können bei verschiedenen Drücken betrieben werden, und in den Verbindungsströmen zwischen den beiden Kolonnen können nur Flüssigkeiten gefördert werden.

Der Reaktoraustrag der ersten Kolonne (K200) kann flüssig oder dampfförmig, bevorzugt dampfförmig in den Abtriebsteil oder den Sumpfbereich, bevorzugt direkt in den Sumpfbereich zugeführt werden.

Die Einspeisung des basischen Amins/Imins kann in die zweite Kolonne K300, bevorzugt in die dritte Kolonne K400 erfolgen, zusätzlich aber auch in die sechste Kolonne K500.

Die Erfindung wird durch die nachstehenden Beispiele für den Aminzusatz näher erläutert.

### Vergleichsbeispiel

1,0 g/h einer Zusammensetzung aus 69,8 Gew.-% Trioxan, 292 Gew.-ppm Ameisensäure und einem Rest aus Formaldehyd und Wasser wurde einer Destillationskolonne zugeführt, die bei einer Temperatur von ca. 182 °C und einem Kopf-Druck von 5,5 bar betrieben wurde. Über Kopf wurden 0,91 g/h einer Zusammensetzung aus 59,7 Gew.-% Trioxan, 501 Gew.-ppm Ameisensäure mit Formaldehyd und Wasser als Rest erhalten, während im Sumpf 0,09 kg/h einer Zusammensetzung aus 99,58 Gew.-% Trioxan und 4200 Gew.-ppm Ameisensäure erhalten wurden. Die Trioxan-Zersetzung betrug 9,3%.

### Beispiel 1

1,0 kg/h einer Zusammensetzung aus 65,8 Gew.-% Trioxan, 350 Gew.-ppm Ameisensäure und einem Rest aus Formaldehyd und Wasser wurden einer Destillationskolonne zugeführt, die bei einem Kopf-Druck von 5,5 bar und einer Temperatur von ca. 182 °C betrieben wurde. Zudem wurden 0,3 ml/h Diazabicycloumdecen dem Feed zugemischt. Über Kopf wurden 0,84 kg/h einer Zusammensetzung aus 58,1 Gew.-% Trioxan, 603 Gew.-ppm Ameisensäure mit einem Rest von Formaldehyd und Wasser erhalten, während im Sumpf 0,16 kg/h einer Zusammensetzung aus 99,99 Gew.-% Trioxan und 100 Gew.-ppm Ameisensäure erhalten wurden. Die Trioxan-Zersetzung betrug 1,5%. Der Sumpfaustrag konnte in einem weiteren Verdampfer in Trioxan über Kopf und Ameisensäure-Amin-Salz im Sumpf aufgetrennt werden, wobei das Salz nach Zersetzung des Formeats zugeführt werden konnte, als Amin.

Die Beispiele zeigen, dass die Trioxan-Zersetzung wirkungsvoll vermindert werden konnte und Ausbeute und Reinheit des Trioxans signifikant verbessert wurden.

Der Aminzusatz ist insbesondere in der erfindungsgemäßen dreistufigen Destillationssequenz vorteilhaft.

## Patentansprüche

1. Verfahren zur Abtrennung von Trioxan aus einem Einsatzstrom I aus Formaldehyd, Trioxan und Wasser, bei dem
a) ein Einsatzstrom I, der als Hauptkomponente Formaldehyd und als Nebenkomponenten Trioxan und Wasser enthält, bereitgestellt wird,
b) der Einsatzstrom I, ein Rückführstrom V und ein Rückführstrom VII, der als Hauptkomponente Formaldehyd und als Nebenkomponenten Wasser und Trioxan enthält, in eine erste Destillationsstufe eingespeist und bei einem Druck von 0,1 bis 2,5 bar destilliert werden, wobei ein Strom II, der als Hauptkomponente Formaldehyd und als Nebenkomponente Wasser enthält, und ein Strom III, der als Hauptkomponente Trioxan und als Nebenkomponenten Wasser und Formaldehyd enthält, und ein Strom X, der Wasser, Trioxan und Formaldehyd enthält, erhalten werden,
c) der Strom III, gegebenenfalls nach Abtrennung von Leichtsiedern aus dem Strom III in einer Leichtsieder-Abtrennstufe, in einer zweiten Destillationsstufe bei einem Druck von 0,2 bis 17,5 bar destilliert wird, wobei der Druck in der zweiten Destillationsstufe um 0,1 bis 15 bar höher als der Druck in der ersten Destillationsstufe ist, wobei ein Strom IV, der im Wesentlichen aus Trioxan besteht, und der Rückführstrom V, der als Hauptkomponente Trioxan und als Nebenkomponenten Wasser und Formaldehyd enthält, erhalten wird,
c1) der Strom IV in mindestens einer weiteren Trioxan-Destillationsstufe bei einem Kopfdruck von 0,5 bis 2 bar aufgereinigt wird, wobei aufgereinigtes Trioxan als Seitenabzugsstrom XII im Verstärkungsteil der Kolonne gewonnen wird,
d) der Strom X und gegebenenfalls ein Strom IX, der als Hauptkomponente Wasser enthält und bei der Aufkonzentrierung von wässriger Formaldehydlösung erhalten wird, in eine dritte Destillationsstufe eingespeist und bei einem Druck von 1 bis 10 bar destilliert wird, wobei ein Strom VI, der im Wesentlichen aus Wasser besteht, und ein Rückführstrom VII, der als Hauptkomponente Formaldehyd und als Nebenkomponenten Wasser und Trioxan enthält, erhalten werden.

2. Verfahren nach Anspruch 1, **dadurch gekennzeichnet, dass** auf Stufe c1) folgend eine zweite Trioxandestillation des Stroms XII als Stufe c2) durchgeführt wird, bei einem Kopfdruck im Bereich von 0,5 bis 2,0 bar, wobei nochmals aufgereinigtes Trioxan als Seitenabzugsstrom im Verstärkungsteil der Kolonne gewonnen wird.

3. Verfahren nach Anspruch 1 oder 2, **dadurch gekennzeichnet, dass** in den Destillationsstufen c1) und c2) der Strom IV bzw. der Strom XII in den Sumpfbereich der Destillationsstufen geführt werden.

4. Verfahren nach einem der Ansprüche 1 bis 3, **dadurch gekennzeichnet, dass** man dem der zweiten und/oder dritten Destillationsstufe zugeführten, Formaldehyd, Trioxan, Wasser und Ameisensäure enthaltenden Gemisch vor oder während der Destillation mindestens ein tertiäres Amin und/oder ein Imin oder ein Gemisch davon, das die Ameisensäure deprotonieren und in ein Salz überführen kann, in einer katalytischen Menge oder in einer Menge zusetzt, die zur Salzbildung mit der gesamten Ameisensäurenmenge ausreicht, und das gebildete Ameisensäure-Amin-Salz in einer Flüssigphase im Destillationssumpf ausschleust.

5. Verfahren nach Anspruch 4, **dadurch gekennzeichnet, dass** man das Amin in einem molaren Überschuss gegenüber der Ameisensäure zusetzt, so dass das Trioxan basisch stabilisiert wird.

6. Verfahren nach Anspruch 4 oder 5, **dadurch gekennzeichnet, dass** das tertiäre Amin ausgewählt ist aus Tri-C₁₋₃-Alkylaminen, cyclischen oder bicyclischen aliphatischen tertiären Aminen, Imidazol oder Pyridin, vorzugsweise aus Diazabicycloundecen (DBU) und Triethylendiamin (TEDA, DABCO).

7. Verfahren nach einem der Ansprüche 4 bis 6, **dadurch gekennzeichnet, dass** das mit dem Destillationssumpf ausgeschleuste Ameisensäure-Amin-Salz einer weiteren Destillation unterworfen wird, in der es aus dem Destillationssumpf ausgeschleust wird, und/oder das ausgeschleuste Ameisensäure-Amin-Salz unter Erwärmung zersetzt wird, wobei das tertiäre Amin zurückgewonnen wird und in das Verfahren zurückgeführt werden kann.

8. Verfahren nach einem der Ansprüche 1 bis 7, **dadurch gekennzeichnet**, das zwischen der ersten und der zweiten Destillationsstufe eine Leichtsieder-Abtrennstufe durchgeführt wird, in der aus dem Strom III Leichtsieder, ausgewählt aus der Gruppe bestehend aus Methylformiat, Methylal, Dimethoxydimethylether und Methanol, abgetrennt werden.

9. Verfahren nach einem der Ansprüche 1 bis 8, **gekennzeichnet durch** die nachstehende Zusammensetzung der Ströme I - VII und X:
Strom I: 40 bis 80 Gew.-% Formaldehyd, 20 bis 59 Gew.-% Wasser, 1 bis 30 Gew.-% Trioxan;
Strom II: 55 bis 85 Gew.-% Formaldehyd, 15 bis 45 Gew.-% Wasser, 0 bis 5 Gew.-% Trioxan;
Strom III: 3 bis 20 Gew.-% Formaldehyd, 10 bis 30 Gew.-% Wasser, 60 bis 75 Gew.-% Trioxan;
Strom IV: 95 bis 100 Gew.-% Trioxan, 0 bis 5 Gew.-% Wasser und Neben- kom ponenten;
Strom V: 5 bis 20 Gew.-% Formaldehyd, 15 bis 35 Gew.-% Wasser, 50 bis 75 Gew.-% Trioxan;
Strom VI: 0 bis 1 Gew.-% Formaldehyd, 99 bis 100 Gew.-% Wasser;
Strom VII: 10 bis 55 Gew.-% Formaldehyd, 5 bis 50 Gew.-% Wasser, 5 bis 55 Gew.-% Trioxan;
Strom X: 10 bis 50 Gew.-% Formaldehyd, 10 bis 50 Gew.-% Wasser, 3 bis 40 Gew.-% Trioxan;
wobei die Ströme I, III, V und VII noch bis zu 15 Gew.-% Leichtsieder, ausgewählt aus der Gruppe bestehend aus Methylformiat, Methylal, Dimethoxydimethylether und Methanol, enthalten können.

10. Verfahren zur Herstellung von Trioxan aus einer wässrigen Formaldehydlösung, bei dem ein Strom XI aus einer wässrigen Formaldehydlösung einer Trioxan-Synthesestufe zugeführt und unter sauren Bedingungen umgesetzt wird, wobei der Strom I erhalten wird, und aus dem Strom I nach dem Verfahren gemäß einem der Ansprüche 1 bis 9 Trioxan abgetrennt wird und wobei der Strom XI aus einem Strom VIII aus einer wässrigen Formaldehydlösung niedrigerer Formaldehyd-Konzentration durch Aufkonzentrieren in einem Verdampfer erhalten werden kann.

## Claims

1. A process for separating trioxane from a feed stream I comprising formaldehyde, trioxane and water, wherein
a) a feed stream I comprising formaldehyde as main component and trioxane and water as secondary components is provided,
b) the feed stream I, a recycle stream V and a recycle stream VII comprising formaldehyde as main component and water and trioxane as secondary components are fed into a first distillation stage and distilled at a pressure of from 0.1 to 2.5 bar to give a stream II comprising formaldehyde as main component and water as secondary component and a stream III comprising trioxane as main component and water and formaldehyde as secondary components and a stream X comprising water, trioxane and formaldehyde,
c) the stream III is, optionally after removal of low boilers from the stream III in a low boiler removal stage, distilled in a second distillation stage at a pressure of from 0.2 to 17.5 bar, with the pressure in the second distillation stage being from 0.1 to 15 bar higher than the pressure in the first distillation stage, to give a stream IV consisting essentially of trioxane and the recycle stream V comprising trioxane as main component and water and formaldehyde as secondary components,
c1) the stream IV is purified in at least one further trioxane distillation stage at a pressure at the top of from 0.5 to 2 bar to give purified trioxane as side offtake stream XII in the enrichment section of the column,
d) the stream X and optionally a stream IX which comprises water as main component and is obtained in the concentration of aqueous formalydehyde solution are fed into a third distillation stage and distilled at a pressure of from 1 to 10 bar to give a stream VI consisting essentially of water and a recycle stream VII comprising formaldehyde as main component and water and trioxane as secondary components.

2. The process according to claim 1, wherein stage c1) is followed by a second trioxane distillation of the stream XII as stage c2) at a pressure at the top in the range from 0.5 to 2.0 bar to give further purified trioxane as side offtake stream in the enrichment section of the column.

3. The process according to claim 1 or 2, wherein, in the distillation stages c1) and c2), the stream IV and the stream XII, respectively, are fed into the bottom region of the distillation stages.

4. The process according to any of claims 1 to 3, wherein at least one tertiary amine and/or an imine or a mixture thereof which can deprotonate formic acid and convert it into a salt is added to the mixture comprising formaldehyde, trioxane, water and formic acid which is fed to the second and/or third distillation stage in a catalytic amount or in an amount sufficient to form a salt with the entire amount of formic acid before or during the distillation and the formic acid-amine salt formed is discharged in a liquid phase in the distillation bottoms.

5. The process according to claim 4, wherein the amine is added in a molar excess over the formic acid so that the trioxane is base-stabilized.

6. The process according to claim 4 or 5, wherein the tertiary amine is selected from among tri-C₁₋₃-alkylamines, cyclic or bicyclic aliphatic tertiary amines, imidazole and pyridine, preferably from among diazabicycloundecene (DBU) and triethylenediamine (TEDA, DABCO).

7. The process according to any of claims 4 to 6, wherein the formic acid-amine salt discharged with the distillation bottoms is subjected to a further distillation in which it is discharged from the distillation bottoms and/or the formic acid-amine salt discharged is subjected to heating so that the tertiary amine is recovered and can be recirculated to the process.

8. The process according to any of claims 1 to 7, wherein a low boiler removal stage in which low boilers selected from the group consisting of methyl formate, methylal, bis(methoxymethyl) ether and methanol are separated off from the stream III is carried out between the first distillation stage and the second distillation stage.

9. The process according to any of claims 1 to 8, wherein the streams I - VII and X have the following compositions:
Stream I: from 40 to 80% by weight of formaldehyde, from 20 to 59% by weight of water, from 1 to 30% by weight of trioxane;
Stream II: from 55 to 85% by weight of formaldehyde, from 15 to 45% by weight of water, from 0 to 5% by weight of trioxane;
Stream III: from 3 to 20% by weight of formaldehyde, from 10 to 30% by weight of water, from 60 to 75% by weight of trioxane;
Stream IV: from 95 to 100% by weight of trioxane, from 0 to 5% by weight of water and secondary components;
Stream V: from 5 to 20% by weight of formaldehyde, from 15 to 35% by weight of water, from 50 to 75% by weight of trioxane;
Stream VI: from 0 to 1% by weight of formaldehyde, from 99 to 100% by weight of water;
Stream VII: from 10 to 55% by weight of formaldehyde, from 5 to 50% by weight of water, from 5 to 55% by weight of trioxane;
Stream X: from 10 to 50% by weight of formaldehyde, from 10 to 50% by weight of water, from 3 to 40% by weight of trioxane;
with the streams I, III, V and VII being able to further comprise up to 15% by weight of low boilers selected from the group consisting of methyl formate, methylal, bis(methoxymethyl) ether and methanol.

10. A process for preparing trioxane from an aqueous formaldehyde solution, wherein a stream XI of an aqueous formaldehyde solution from a trioxane synthesis stage is fed in and reacted under acid conditions to give the stream I and trioxane is separated from the stream I by the process according to any of claims 1 to 9, where the stream XI can be obtained from a stream VIII of an aqueous formaldehyde solution having a lower formaldehyde concentration by concentration in a vaporizer.

## Revendications

1. Procédé pour la séparation de trioxane à partir d'un flux de départ I constitué de formaldéhyde, de trioxane et d'eau, dans lequel
a) on met à disposition un flux de départ I, qui contient comme composant principal du formaldéhyde et comme composants secondaires du trioxane et de l'eau,
b) on injecte le flux de départ I, un flux de recyclage V et un flux de recyclage VII, qui contient comme composant principal du formaldéhyde et comme composants secondaires de l'eau et du trioxane, dans une première étape de distillation et on distille à une pression de 0,1 à 2,5 bars, avec obtention d'un flux II, qui contient comme composant principal du formaldéhyde et comme composant secondaire de l'eau, et d'un flux III, qui contient comme composant principal du trioxane et comme composants secondaires de l'eau et du formaldéhyde, et d'un flux X, qui contient de l'eau, du trioxane et du formaldéhyde,
c) on distille le flux III, le cas échéant après séparation des substances à bas point d'ébullition du flux III dans une étape de séparation des substances à bas point d'ébullition, dans une deuxième étape de distillation à une pression de 0,2 à 17,5 bars, la pression dans la deuxième étape de distillation étant supérieure de 0,1 à 15 bars à la pression dans la première étape de distillation, avec obtention d'un flux IV, qui est essentiellement constitué de trioxane, et d'un flux de recyclage V, qui contient comme composant principal du trioxane et comme composants secondaires de l'eau et du formaldéhyde,
c1) le flux IV est purifié dans au moins une autre étape de distillation de trioxane à une pression de tête de 0,5 à 2 bars, du trioxane purifié étant obtenu comme flux latéral soutiré XII dans la partie de rectification de la colonne,
d) le flux X et le cas échéant un flux IX, qui contient comme composant principal de l'eau et qui est obtenu lors de la concentration de la solution aqueuse de formaldéhyde, sont injectés dans une troisième étape de distillation et on distille à une pression de 1 à 10 bars, avec obtention d'un flux VI, qui est essentiellement constitué d'eau et d'un flux de recyclage VII, qui contient comme composant principal du formaldéhyde et comme composants secondaires de l'eau et du trioxane.

2. Procédé selon la revendication 1, **caractérisé en ce qu'**on réalise après l'étape c1) une deuxième distillation de trioxane du flux XII en tant qu'étape c2), à une pression de tête dans la plage de 0,5 à 2,0 bars, en obtenant à nouveau du trioxane purifié comme flux latéral soutiré dans la partie de rectification de la colonne.

3. Procédé selon la revendication 1 ou 2, **caractérisé en ce que** dans les étapes de distillation c1) et c2), le flux IV ou, selon le cas, le flux XII sont guidés dans la zone du fond des étapes de distillation.

4. Procédé selon l'une quelconque des revendications 1 à 3, **caractérisé en ce qu'**on ajoute au mélange introduit dans la deuxième et/ou la troisième étape de distillation, contenant du formaldéhyde, du trioxane, de l'eau et de l'acide formique, avant ou pendant la distillation, au moins une amine tertiaire et/ou une imine ou un mélange de celles-ci, qui peut déprotoner l'acide formique et le transformer en sel, en une quantité catalytique ou en une quantité qui suffit pour la formation de sel avec la quantité totale d'acide formique, et on évacue le sel d'amine de l'acide formique formé dans une phase liquide dans le fond de la distillation.

5. Procédé selon la revendication 4, **caractérisé en ce qu'**on ajoute l'amine en un excès molaire par rapport à l'acide formique, de manière telle que le trioxane est stabilisé de manière basique.

6. Procédé selon la revendication 4 ou 5, **caractérisé en ce que** l'amine tertiaire est choisie parmi les tri-C₁-₃-alkylamines, les amines tertiaires aliphatiques cycliques ou bicycliques, l'imidazole ou la pyridine, de préférence parmi le diazabicyclo-undécène (DBU) et la triéthylènediamine (TEDA, DABCO).

7. Procédé selon l'une quelconque des revendications 4 à 6, **caractérisé en ce que** le sel d'amine de l'acide formique évacué avec le fond de distillation est soumis à une autre distillation dans laquelle il est évacué du fond de distillation et/ou le sel d'amine de l'acide formique évacué est décomposé en chauffant, l'amine tertiaire étant récupérée et pouvant être recyclée dans le procédé.

8. Procédé selon l'une quelconque des revendications 1 à 7, **caractérisé en ce qu'**on réalise, entre la première et la deuxième étape de distillation, une étape de séparation des substances à bas point d'ébullition, dans laquelle on élimine du flux III des substances de bas point d'ébullition choisies dans le groupe constitué par le formiate de méthyle, le méthylal, le diméthoxydiméthyléther et le méthanol.

9. Procédé selon l'une quelconque des revendications 1 à 8, **caractérisé par** la composition suivante des flux I-VII et X :
Flux 1 : 40 à 80% en poids de formaldéhyde, 20 à 59% en poids d'eau, 1 à 30% en poids de trioxane ;
Flux II : 55 à 85% en poids de formaldéhyde, 15 à 45% en poids d'eau, 0 à 5% en poids de trioxane ;
Flux III : 3 à 20% en poids de formaldéhyde, 10 à 30% en poids d'eau, 60 à 75% en poids de trioxane ;
Flux IV : 95 à 100% en poids de trioxane, 0 à 5% en poids d'eau et des composants secondaires ;
Flux V : 5 à 20% en poids de formaldéhyde, 15 à 35% en poids d'eau, 50 à 75% en poids de trioxane ;
Flux VI : 0 à 1% en poids de formaldéhyde, 99 à 100% en poids d'eau ;
Flux VII : 10 à 55% en poids de formaldéhyde, 5 à 50% en poids d'eau, 5 à 55% en poids de trioxane ;
Flux X : 10 à 50% en poids de formaldéhyde, 10 à 50% en poids d'eau, 3 à 40% en poids de trioxane ;
les flux I, III, V et VII pouvant encore contenir jusqu'à 15% en poids de substances à bas point d'ébullition, choisies dans le groupe formé par le formiate de méthyle, le méthylal, le diméthoxydiméthyléther et le méthanol.

10. Procédé pour la préparation de trioxane à partir d'une solution aqueuse de formaldéhyde, dans lequel on introduit un flux XI d'une solution aqueuse de formaldéhyde dans une étape de synthèse de trioxane et on transforme dans des conditions acides, avec obtention du flux I et on sépare du flux I, selon le procédé selon l'une quelconque des revendications 1 à 9, du trioxane et où le flux XI peut être obtenu à partir d'un flux VIII à partir d'une solution aqueuse de formaldéhyde de faible concentration en formaldéhyde par concentration dans un évaporateur.
